# EUROPÄISCHE PATENTSCHRIFT

(11) **EP 0 631 763 B1**
(45) Veröffentlichungstag und Bekanntmachung des Hinweises auf die Patenterteilung: **19.03.1997**
(21) Anmeldenummer: 93810466.8
(22) Anmeldetag: 01.07.1993
(51) Int. Cl.: A61F 2/28, A61L 27/00

(54) **Füllkörper aus Metall für Knochenhohlräume**
Metallic filler for bone cavities
Corps de remplissage en métal pour cavités osseuses

(43) Veröffentlichungstag der Anmeldung: 04.01.1995
(73) Patentinhaber: Sulzer Orthopädie AG, 6340 Baar (CH)
(72) Erfinder: Koller, Hansjürg, CH-8400 Winterthur (CH)
(74) Vertreter: Trieblnig, Adolf

(56) Entgegenhaltungen:
- EP-A- 0 220 427
- EP-A- 0 338 976
- EP-A- 0 366 018
- EP-A- 0 526 682
- DE-A- 2 910 627
- " Grosses Textil-Lexikon" S. 520, 521 zum Wort " Gewirke" Deutsche Verlags-Anstalt Stuttgart, herausgegeben von Paul-August Koch und Günther Satlow

## Beschreibung

Die Erfindung bezieht sich auf einen Füllkörper aus Metall für Knochenhohlräume gemäss dem Oberbegriff von Anspruch 1. Die Erfindung bezieht sich weiter auf ein Verfahren zur Herstellung von Füllkörpern.

Bei Operationen am Knochen, insbesondere bei der Implantation von Hüftgelenken, kann es erforderlich sein, in einem Knochenhohlraum einen Füllkörper einzubringen, um dem Knochen eine zusätzliche Stütze zu verleihen, oder um den Knochenhohlraum zum Beispiel gegen den Markraum hin abzugrenzen. Der Füllkörper muss hohen Anforderungen genügen. So muss der Füllkörper biokompatibel sein und die Eigenschaft aufweisen, dass sich während und nach der Operation keine Einzelfragmente vom Füllkörper lösen und sich irgendwo in unkontrollierbarer Weise im Körper ablagern. Aus der US-PS 3,906,550 ist ein Füllkörper bekannt, der aus kurzen, wirr gekrümmten, metallischen Fasern besteht, die in einem Sinterprozess zu einem porösen Füllkörper zusammengebacken wurden. Ein sehr gut körperverträgliches Material ist Titan, dessen Verarbeitung jedoch äusserst anspruchsvoll ist. Wird Titan während dem Verarbeitungsprozess zur Formgebung erwärmt, zum Beispiel in einem Sinterprozess, so besteht die Gefahr der Versprödung. Bei einem mit einem solchen formgebenden Verfahren bearbeiteten Füllkörper besteht somit die Gefahr sich lösender Einzelfragmente. Eine Erwärmung während der Formgebung kann durch eine spanende Fertigung vermieden werden. Dabei besteht jedoch die Gefahr, dass sich Kleinstspäne bilden.

Es ist Aufgabe der Erfindung, einen Füllkörper für Knochenhohlräume herzustellen, der bezüglich dem Ablösen von Einzelfragmenten verbesserte Eigenschaften aufweist, wobei dessen Porosität beeinflussbar ist.

Diese Aufgabe wird erfingungsgemäss gelöst gemäss den Merkmalen von Anspruch 1, beziehungsweise mit einem Herstellungsverfahren gemäss den Merkmalen von Anspruch 5. Die Unteransprüche beziehen sich auf weitere vorteilhafte Ausführungsformen der Erfindung.

Der Füllkörper besteht aus einem Gewirk oder Gestrick aus Draht, wobei der Füllkörper seine Form durch plastische Deformation des Gewirks erhält.

Die Vorteile der Erfindung sind darin zu sehen, dass sich aus dem Füllkörper keine Einzelfragmente mehr lösen. Der Füllkörper besteht üblicherweise aus einem Gewirk, das mit einem einzigen oder wenigen Drähten hergestellt ist. Die plastische Verformung des Gewirks bewirkt einen Verbund verstrickter Maschen, derart, dass die einzelnen Maschen nicht mehr lösbar sind. Die plastische Verformung des Gewirks ist vorteilhafterweise derart auszuführen, dass sich die Drähte plastisch verformen, jedoch nicht brechen. Der Füllkörper weist nach der Verformung Poren auf. Über die Presskraft sowie die Wahl des Drahtdurchmessers oder des Drahtquerschnittes kann die Porösität des Füllkörpers beziehungsweise die Festigkeit des Füllkörpers in weiten Grenzen variiert werden. Ist der Füllkörper aus Reintitan zu fertigen, so werden dazu entsprechende Drähte aus Reintitan verwendet. Ein Vorteil ist darin zu sehen, dass es zur Formgebung des Füllkörpers aus Reintitan weder einer Erwärmung noch eines spanenden Fertigungsprozess bedarf. Ein weiterer Vorteil ist darin zu sehen, dass durch die Formgebung des Füllkörpers mittels plastischer Verformung eine Vielzahl möglicher Formen herstellbar sind, insbesondere dann, wenn der Prozess der plastischen Verformung in mehreren einzelnen Schritten ausgeführt wird.

Im folgenden wird die Erfindung anhand eines Ausführungsbeispieles beschrieben. Es zeigen:
- Fig. 1a: Ein zylinderförmiges Gewirk;
- Fig. 1b: eine Detailansicht des Fadenverlaufs im Gewirk;
- Fig. 2a: eine Vorrichtung zur Formgebung vor dem Pressen;
- Fig. 2b: eine Vorrichtung zur Formgebung nach dem Pressen;
- Fig. 2c: einen Füllkörper.

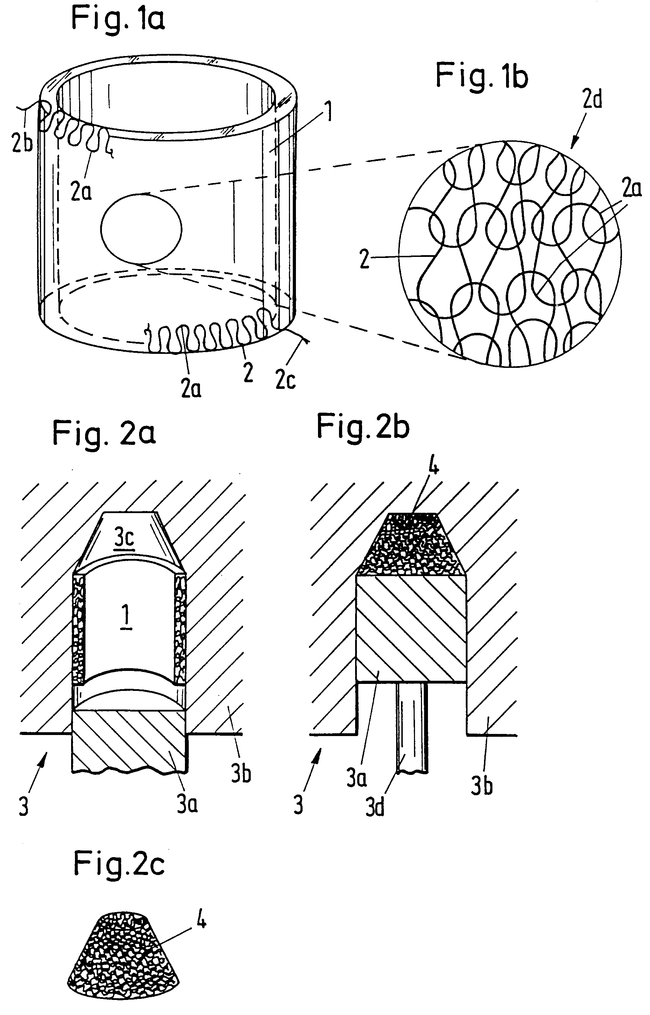

Gewirke sind Flächengebilde, die aus einem oder mehreren Fadensystemen durch Maschenbildung auf Wirk- oder Strickmaschinen hergestellt werden. Fig. 1b zeigt einen Ausschnitt eines Gewirkes 2d beziehungsweise eines Gestrickes dessen Maschen 2a aus einem Draht 2 bestehen. Die Grösse der Maschen 2a und somit der Biegungsradius des Drahtes 2 ist bei der Herstellung in weiten Grenzen variierbar. Gewirke 2d besitzen insbesondere in Breitenrichtung, dies heisst bezüglich Fig. 1b in horizontaler Richtung, eine hohe Dehnung und Elastizität. Weiter ergibt sich durch die Maschenstruktur ein grosses Porenvolumen.
In Fig. 1a ist ein auf einer Rundstrickmaschine hergestellter, rohrförmiger Körper 1 aus Draht 2, vorzugsweise aus Titandraht, dargestellt. Das ganze Gebilde 1 besteht im vorliegenden Ausführungsbeispiel aus einem einzigen Draht 2, mit zwei Drahtenden 2b, 2c. Das Gestrick ist relativ grobmaschig und locker, um den Draht 2 durch Stricken verarbeiten zu können und einen Rohling 1 in Form eines Hohlzylinders herzustellen. Für Reintitan hat sich ein Drahtdurchmesser zwischen 0,1 mm und 0,8 mm als besonders vorteilhaft erwiesen. In einem weiteren Verfahrensschritt, der in den Figuren 2a-2c dargestellt ist, wird aus dem Rohling 1 durch Pressvorgänge ein Füllkörper 4 geformt. Fig. 2a zeigt ein Presswerkzeug 3 mit Pressform 3b, Stempel 3a und Hohlraum 3c. Der rohrförmige Körper 1 ist im Hohraum 3c eingelegt, und wird, wie in Fig. 2b dargestellt, durch den in den Hohlraum3c einfahrenden Stempel 3a plastisch verformt, sodass der Körper 1 die durch die Pressform 3b vorgegebene Form annimmt, und somit zu einem Körper 4 transformiert wird. Vorteilhafterweise werden vor dem Verformen des Körpers 1 die Drahtenden 2b, 2c derart in den Körper 1 eingelegt, dass die Enden nach dem Umformvorgang in das Innere des transformierten Körpers 4 zu liegen kommen. Fig. 2c zeigt einen kegelstumpfförmigen Körper 4, der als Füllkörper 4 in Knochenhohlräume, beispielsweise als Markraumsperre eingesetzt werden kann. Die plastische Umformung des rohrförmigen Körpers 1 kann natürlich über mehrere Schritte und mit unterschiedlichen Werkzeugen erfolgen, sodass erst nach mehreren Arbeitsschritten ein Füllkörper 4 vorliegt, dessen Form sich zum Beispiel für den Einsatz in Knochenhohlräumen eignet. Mit der plastischen Verformung wird natürlich auch die Porosität des Füllkörpers 4 beeinflusst. Das Herstellungsverfahren erlaubt eine grosse Vielfalt von Formen des Füllkörpers 4 in unterschiedlichster Porosität herzustellen. So kann das Maschengebilde 1 auf unterschiedlichste Weise gestrickt oder gewirkt sein, so auch als ebenes, flächiges Gebilde, das auch Ausnehmungen aufweisen kann. Das Gewirke kann mit mehreren Fäden oder Drähten hergestellt sein, wobei diese auch unterschiedliche Durchmesser oder Querschnitte aufweisen können.

## Patentansprüche

1. Füllkörper aus Metall für Knochenhohlräume, der aus einem Gewirk (2d) oder Gestrick mit mindestens einem Draht (2) besteht, wobei das Gewirk (2d) oder Gestrick durch plastische Deformation auf eine im Vergleich zu seinen ursprünglichen Dimensionen wesentlich kleinere Raumform zusammengestaucht ist.

2. Füllkörper nach Anspruch 1, dadurch gekennzeichnet, dass dieser aus einem einzigen Draht (2) besteht.

3. Füllkörper nach Anspruch 1 oder 2, dadurch gekennzeichnet, dass der Draht (2) einen Durchmesser im Bereich zwischen 0,1 mm und 0,8 mm aufweist.

4. Füllkörper nach einem der Ansprüche 1 bis 3, dadurch gekennzeichnet, dass der Draht (2) aus Reintitan besteht.

5. Füllkörper nach einem der Ansprüche 1 bis 4, dadurch gekennzeichnet, dass der Füllkörper (4) eine einfach zusammenhängende Form aus der Gruppe zylinderförmig, kegelförmig, kegelstumpfförmig oder kubisch aufweist.

6. Verfahren zur Herstellung eines Füllkörpers aus Metall für Knochenhohlräume, bei dem mit einer Wirkmaschine aus einem oder mehreren Drähten (2) ein Gewirk (2d) hergestellt wird, und das Gewirk (2d) danach einer Pressvorrichtung (3) zugeführt und durch plastische Deformation auf eine im Vergleich zu seinen ursprünglichen Dimensionen wesentlich kleinere Raumform zusammengestaucht wird, sodass die Form des Füllkörpers (4) durch plastische Verformung erzeugt wird.

7. Verfahren nach Anspruch 6, dadurch gekennzeichnet, dass das Gewirk (2d) auf einer Rundstrickmaschine als rohrförmiger Körper (1) hergestellt wird.

8. Verfahren nach Anspruch 6 oder 7, dadurch gekennzeichnet, dass die Enden des Drahtes (2), die das Gewirk (2d) aufweist, vor dem Pressvorgang in das Gewirk (2d) eingelegt werden.

9. Verfahren nach einem der Ansprüche 6 bis 8, dadurch gekennzeichnet, dass die Porosität des Füllkörpers durch den Drahtdurchmesser und/oder durch den Drahtquerschnitt und/oder durch den Pressdruck bestimmt wird.

## Claims

1. A filling member made from metal for bone cavities, which is made from a knitted fabric (2d) having at least one wire (2), with the knitted fabric (2d) being compressed by plastic deformation to a three-dimensional shape which is substantially smaller when compared with its original dimensions.

2. A filling member according to Claim 1,
**characterised in that** it is made of a single wire (2).

3. A filling member according to Claim 1 or 2,
**characterised in that** the wire (2) has a diameter in the range between 0.1 mm and 0.8 mm.

4. A filling member according to one of Claims 1 to 3,
**characterised in that** the wire (2) is made from pure titanium.

5. A filling member according to one of Claims 1 to 4,
**characterised in that** the filling member (4) comprises an easily cohesive shape which may be cylindrical, conical, in the shape of a truncated cone or cubic.

6. A process for the manufacture of a filling member made from metal for bone cavities, in which a knitted fabric (2d) is manufactured with a knitting machine from one or more wires (2), and the knitted fabric (2d) is subsequently supplied to a pressing device (3) and is compressed by plastic deformation to a substantially smaller three-dimension shape in comparison with its original dimensions, so that the shape of the filling member (4) is produced by plastic deformation.

7. A process according to Claim 6,
**characterised in that** the knitted fabric (2d) is manufactured on a round knitting machine as a tubular body (1).

8. A process according to Claim 6 or 7,
**characterised in that** the ends of the wire (2), which the knitted fabric (2d) has, are inserted into the knitted fabric (2d) before the pressing operation.

9. A process according to one of Claims 6 to 8,
**characterised in that** the porosity of the filling member is determined by the wire diameter and/or by the wire cross section and/or by the pressure.

## Revendications

1. Corps de remplissage en métal, pour des cavités osseuses, constitué d'un tissu (2d) ou d'un tricot comportant au moins un fil métallique (2), le tissu (2d) ou le tricot étant comprimé par refoulement, par déformation plastique, jusqu'à atteindre une forme spatiale notablement plus petite par rapport à ses dimensions d'origine.

2. Corps de remplissage selon la revendication 1, caractérisé en ce qu'il est constitué en un fil métallique (2) unique.

3. Corps de remplissage selon la revendication 1 ou 2, caractérisé en ce que le fil métallique (2) a un diamètre situé dans la plage comprise entre 0,1 mm et 0,8 mm.

4. Corps de remplissage selon l'une des revendications 1 à 3, caractérisé en ce que le fil métallique (2) est en titane pur.

5. Corps de remplissage selon une des revendications 1 à 4, caractérisé en ce que le corps de remplissage (4) a une forme cohérente simple, choisie dans le groupe des formes cylindrique, conique, tronconique ou cubique.

6. Procédé de fabrication d'un corps de remplissage en métal pour des cavités osseuses, dans lequel on fabrique à l'aide d'une machine à tisser, à partir d'un ou plusieurs fils (2), un tissu (2d) et le tissu (2d) étant amené à un dispositif de pressage (3) et écrasé par refoulement, par déformation plastique, jusqu'à à atteindre une forme spatiale notablement plus petite par rapport à ses dimensions d'origine, de manière que la forme du corps de remplissage (4) soit produite par la déformation plastique.

7. Procédé selon la revendication 6, caractérisé en ce que le tissu (2d) est fabriqué sur un métier à tricoter circulaire, sous la forme d'un corps (1) tubulaire.

8. Procédé selon la revendication 6 ou 7, caractérisé en ce que les extrémités du fil métallique (2) que présente le tissu (2d) sont insérées dans le tissu (2d), avant le déroulement du processus de pressage.

9. Procédé selon l'une des revendications 6 à 8, caractérisé en ce que la porosité du corps de remplissage est déterminée par le diamètre du fil métallique et/ou la section transversale du fil oblique et/ou par la pression de pressage.
